# FASCICULE DE BREVET EUROPEEN

(11) **EP 1 797 848 B1**
(45) Date de publication et mention de la délivrance du brevet: **09.03.2011**
(21) Numéro de dépôt: 06125638.4
(22) Date de dépôt: 07.12.2006
(51) Int. Cl.: A61F 7/02, A61F 7/08

(54) **Procédé de traitement cosmétique ou dermatologique et dispositifs pour la mise en oeuvre d'un tel procédé**
Kosmetische oder dermatologische Behandlungsmethoden und Geräte
Cosmetic or dermatologic treatment process and devices therefor

(30) Priorité: 09.12.2005 FR 0553822
(43) Date de publication de la demande: 20.06.2007
(73) Titulaire: L'Oréal, 75008 Paris (FR)
(72) Inventeur: Gueret, Jean-Louis H., 75016, Paris (FR)
(74) Mandataire: Leray, Noelle

(56) Documents cités:
- EP-A- 0 779 043
- EP-A- 1 334 921
- EP-A- 1 593 319
- EP-A1- 0 483 968
- FR-A- 2 849 753
- US-A- 4 291 685
- US-A- 5 630 959
- US-A1- 2005 220 828

## Description

La présente invention concerne le traitement cosmétique ou dermatologique de la peau avec apport de chaleur, voire le traitement des autres matières kératiniques telles que les cheveux, par exemple.

La demande de brevet européen EP 1 593 319 divulgue un procédé de traitement cosmétique, dans lequel un récipient est placé dans un four à micro-ondes pour élever la température d'une composition cosmétique contenue à l'intérieur. La composition ainsi réchauffée est appliquée au moyen d'un applicateur. La demande FR 2 849 753 divulgue également un procédé de traitement cosmétique, dans lequel un support imprégné d'une composition cosmétique est placé dans un four à micro-ondes pour élever la température de la composition cosmétique.

Par ailleurs, il existe des masques pouvant être placés dans un four à micro-ondes afin de permettre une application de chaleur sur le visage. Un tel masque est commercialisé sous la dénomination COLDHOT^{®} par la société 3M et son utilisation à chaud peut se faire pour améliorer l'efficacité de certains produits cosmétiques.

La possibilité d'introduire une composition cosmétique dans un four à micro-ondes avant l'application n'est pas sans poser des problèmes de formulation.

En effet, certaines compositions cosmétiques sont susceptibles de s'altérer lorsque chauffées, n'étant pas stables à la chaleur.

De plus, lorsqu'une faible quantité de composition est utilisée localement, l'effet de chaud est de courte durée à cause du refroidissement de la composition au contact de l'air et de la peau.

Enfin, certains dispositifs de distribution sont prévus pour fonctionner avec des compositions présentant des rhéologies particulières et peuvent ne plus fonctionner correctement lorsque la viscosité de la composition est modifiée suite à un changement de température.

Le brevet US3752155 décrit une bille métallique pouvant être chauffée au moyen d'un appareil comportant des résistances chauffantes au contact desquelles est amenée la bille.

La demande de brevet US2003/0100936 décrit un rouleau dans lequel peut être introduit un liquide chauffé.

La demande WO 2005/087043 décrit un procédé pour traiter les tissus kératiniques dans lequel un sachet contenant une composition est chauffé dans une enceinte pourvue de moyens de chauffage. Le sachet est notamment réalisé à partir de films plastiques pouvant être métallisés.

La demande de brevet EP 1 462 025 décrit une brosse à mascara associée à un réservoir de produit dans lequel est prévue une paroi chauffante pour chauffer la brosse à mascara chargée de produit.

Le brevet US 5,775,344 décrit une brosse à mascara comportant une tige chauffante.

Le brevet US 5,856,653 décrit une enceinte de chauffage dans laquelle peut être introduit un récipient contenant un produit cosmétique en vue de le chauffer. L'enceinte comporte des moyens de chauffage qui chauffent un fluide prévu dans la paroi de l'enceinte.

Le document US-A-4 291 685 décrit un applicateur cosmétique permettant de chauffer, puis de délivrer une solution cosmétique.

Il existe donc un besoin pour pouvoir bénéficier d'un effet de chaud durable lors de l'application d'une composition cosmétique sans être confronté aux inconvénients des procédés et dispositifs connus.

L'invention vise à proposer un procédé de traitement des matières kératiniques permettant l'utilisation de chaleur, qui soit compatible avec une large diversité de compositions cosmétiques.

L'invention est comme décrite dans le jeu de revendication ci-joint.

L'invention a trait, selon l'un de ses aspects, à un procédé de traitement cosmétique, non thérapeutique, d'au moins une région du corps humain, comportant les étapes suivantes :
a) réchauffer à une température supérieure ou égale à 30 °C, un applicateur, puis
b) charger l'applicateur ainsi réchauffé avec une composition cosmétique à une température plus proche de la température ambiante que celle de l'applicateur et ayant une différence d'au moins 5 °C avec cette dernière, et
   appliquer la composition en utilisant l'applicateur, ou
c) avant ou après l'étape a), appliquer sur la région à traiter au moins une composition cosmétique et après l'étape a) amener l'applicateur ainsi réchauffé au contact de la région à traiter.

La composition est par exemple appliquée sur la peau ou les cheveux juste avant d'amener l'applicateur à son contact. La composition peut encore avoir été appliquée plus d'une heure avant, voire la veille par exemple, selon le traitement à effectuer.

L'invention peut permettre de traiter une région du corps humain avec apport de chaleur au moyen d'une composition cosmétique pouvant être quelconque, puisque n'étant réchauffée le cas échéant qu'au moment de l'utilisation.

Lorsque la composition est prélevée dans un récipient, un risque de dénaturation, sous l'effet de la chaleur, de la composition non utilisée au sein du récipient la contenant peut ainsi être évité.

L'applicateur peut être utilisé comme outil de massage.

La durée de contact interrompu sur la peau et les cheveux est par exemple comprise entre 0,5 s et 30 mn, voire plus courte ou plus longue selon le traitement effectué.

L'apport de chaleur peut renforcer l'action de la composition, par exemple en préparant l'épiderme à son action, en dilatant les pores, en créant une hyperémie, en activant la circulation et/ou en favorisant la pénétration des actifs. L'applicateur peut apporter un effet relaxant.

L'applicateur peut être utilisé pour exercer, le cas échéant, une action de massage sans craindre de distribuer un excès de composition sur la région traitée, puisque l'applicateur peut être amené au contact de la région traitée sans que son utilisation ne s'accompagne nécessairement de la distribution de composition sur la région traitée.

Le procédé peut comporter seulement l'étape b) ou seulement l'étape c).

La température à laquelle l'applicateur est réchauffé peut être, par exemple, supérieure ou égale à 50 °C et inférieure ou égale à 80 °C.

L'applicateur peut comporter, dans une cavité au moins, un composé, notamment un composé pouvant changer d'état lorsque réchauffé et l'applicateur peut être réchauffé à une température suffisamment élevée pour provoquer ce changement d'état.

Le composé peut être un solide qui se liquéfie par apport de chaleur, par exemple une cire. L'applicateur peut alors restituer la chaleur latente de solidification lorsque ce composé se refroidit.

L'applicateur peut comporter au moins 0,2 cm³ dudit composé.

La région à traiter peut être la peau du visage ou du reste du corps, y compris les muqueuses, ou les cheveux.

Dans un exemple de mise en oeuvre de l'invention, le procédé comporte l'application sur la région à traiter d'un substrat portant la composition, l'applicateur étant amené au contact du substrat ainsi appliqué.

Ce substrat peut comporter un tissé, un non-tissé ou une mousse.

La composition cosmétique peut être prélevée dans un récipient pour être appliquée sur la région à traiter. La composition peut être prélevée au moyen de l'applicateur ou autrement, par exemple avec un doigt ou une spatule ou un dispositif de distribution tel qu'une pompe. Le récipient peut comporter un embout de distribution.

Un premier traitement de la même région ou de régions différentes peut être effectué avec un applicateur porté à température supérieure ou égale à 30 °C et un deuxième traitement de cette ou ces régions peut être effectué avec un applicateur porté à une température inférieure ou égale à 15 °C, ou inversement. Le même applicateur peut être utilisé, le cas échéant. Des applicateurs différents peuvent aussi être utilisés.

La composition cosmétique peut comporter au moins un composé pouvant changer d'état à la température à laquelle l'applicateur est réchauffé et la chaleur apportée par l'applicateur à la composition cosmétique peut être suffisante pour provoquer le changement d'état d'une partie au moins de ce composé.

La composition peut être appliquée sur une ride par exemple et l'applicateur peut provoquer momentanément une fusion du composé au sein de la ride, lorsqu'amené à son contact.

Le composé peut être particulaire. Le composé peut comporter par exemple des particules comportant au moins un polymère ayant une température de fusion inférieure ou égale à la température à laquelle est portée une surface d'application de l'applicateur, et par exemple supérieure ou égale à 30 °C.

L'applicateur peut être encore utilisé, le cas échéant, à température ambiante, si l'utilisateur le souhaite.

La composition peut être contenue dans un récipient fermé lorsque l'applicateur est utilisé.

L'invention a encore pour objet, selon l'un de ses aspects, un ensemble comportant :
- éventuellement une composition cosmétique ou dermatologique contenue dans un récipient ou substrat,
- un applicateur pouvant être séparé du récipient ou substrat et ayant une surface d'application pour traiter une région de peau, comportant au moins un matériau ayant un comportement thermique tel que lorsqu'il est réchauffé dans un four à micro-ondes à une température ne causant pas de lésion thermique à la peau lors du contact de la surface d'application avec la peau pendant 15 s, la surface d'application présente, après cette application une température supérieure ou égale à 30 °C.

L'invention a encore pour objet, selon un autre de ses aspects, un ensemble comportant :
- éventuellement, une composition cosmétique ou dermatologique contenue dans un récipient ou substrat,
- un applicateur ayant une surface d'application destinée à venir au contact de la région à traiter, ayant au moins une cavité contenant un composé, par exemple un composé pouvant changer d'état lorsque réchauffé à une température supérieure ou égale à 30 °C et inférieure ou égale à 80 °C.

La surface d'application peut ne pas être rotative. Cela signifie que la surface d'application n'est pas définie par un élément monté en rotation autour d'un axe de rotation, comme par exemple une bille rotative ou un rouleau rotatif. En utilisant une surface d'application non rotative, on peut appliquer une grande surface ayant une même température relativement élevée sur la région de la peau à traiter, ce qui n'est pas le cas lorsqu'on utilise un élément d'application rotatif.

L'applicateur peut être non métallique. Le composé peut être un liquide.

L'ensemble peut comporter au moins 0,2 cm³ dudit composé, mieux entre 1 et 80 cm³ du composé, notamment entre 5 cm³ et 70 cm³.

L'applicateur peut comporter un matériau relativement dense, par exemple de densité supérieure ou égale à 1,5 g/cm³, définissant au moins partiellement une surface d'application destinée à venir au contact de la région à traiter. Ce matériau peut être du verre ou une pierre, par exemple.

L'applicateur peut comporter un matériau définissant au moins partiellement la surface d'application et présentant une conductivité thermique supérieure ou égale à 1 Wm⁻¹ K⁻¹, mieux 40 Wm⁻¹ K⁻¹ et/ou un matériau de capacité calorifique massique supérieure ou égale à 500 J kg⁻¹ K⁻¹, mieux 1 000 J kg⁻¹ K⁻¹, encore mieux 2 000 J kg⁻¹ K⁻¹. Ce matériau peut être en contact avec le composé contenu dans la cavité, le cas échéant. L'applicateur peut comporter un matériau de chaleur massique supérieure ou égale à 500 J kg⁻¹ K⁻¹. Ce matériau peut être du verre ou une pierre, par exemple.

Une conductivité thermique élevée favorise le transfert de chaleur entre l'applicateur et la région à traiter et permet de bénéficier d'un renouvellement de chaud rapide, tant que les calories emmagasinées par l'applicateur le permettent.

La capacité thermique est par exemple suffisante pour qu'à température ambiante de 20 °C, la surface d'application conserve pendant au moins 10 minutes une température supérieure ou égale à 30 °C lorsque portée initialement à 50 °C (température uniforme).

L'applicateur peut être tel que lorsque réchauffé (de façon uniforme) à 50 °C et appliqué sur la peau, il conserve notamment au niveau de la surface d'application pendant au moins 30 s, mieux 1 mn, encore mieux 15 mn ou 30 mn, une température supérieure ou égale à 30 °C.

La surface d'application peut être définie au moins partiellement par un matériau présentant une inertie thermique supérieure ou égale à 1 000 Jm⁻² K⁻¹ s^{-1/2}, mieux supérieure ou égale à 5 000, encore mieux 10 000 Jm⁻² K⁻¹ s^{-1/2}.

L'inertie thermique caractérise l'aptitude de la surface d'application à conserver sa température lorsqu'exposée périodiquement à un contact avec la peau.

L'inertie thermique est définie par la formule (k.ρ.C)^{1/2}, où k est la conductivité thermique, p la densité volumique et C la capacité calorifique massique.

L'applicateur peut présenter une masse supérieure ou égale à 15 g. Une masse élevée peut permettre d'accroître la capacité thermique.

L'applicateur peut comporter une surface de préhension définie au moins partiellement par un matériau présentant une conductivité thermique inférieure ou égale à 1 Wm⁻¹K⁻¹, mieux 0,5 voire 0,1 Wm⁻¹K⁻¹. Ce matériau peut comporter une matière plastique, par exemple thermoplastique, ou du bois. Ce matériau peut présenter une structure alvéolaire, par exemple.

La composition et l'applicateur peuvent être contenus initialement dans un même emballage.

L'applicateur peut également contenir des matériaux non métalliques mais denses, par exemple de densité supérieure ou égale à 1,1 g/cm³, mieux 1,5 g/cm³, par exemple du sable, du verre ou de la kimberlite.

L'applicateur peut comporter au moins une partie qui est moulée, par exemple par injection ou soufflage, ou qui est usinée.

La cavité précitée peut être réalisée par moulage et/ou par usinage. L'applicateur peut comporter plus d'une cavité contenant le composé pouvant changer d'état.

L'applicateur peut comporter un matériau relativement dense et une matière plastique, un verre et une matière plastique, par exemple.

La surface d'application peut être douce et polie ou en variante, comporter des aspérités ou reliefs tels que des picots.

La surface d'application peut être définie par un matériau dur ou non. Le cas échéant, la surface d'application peut être définie au moins partiellement par une paroi au moins partiellement recouverte d'une membrane élastomère, d'une mousse, d'un flocage, d'un film de matière plastique, d'une éponge, d'un feutre, d'un tissé ou non-tissé. Cette paroi ainsi recouverte est par exemple constituée au moins partiellement par un matériau métallique.

L'applicateur peut être agencé pour se fixer de manière amovible sur le récipient. L'applicateur peut être agencé pour se fixer de manière amovible sur un organe de fermeture du récipient. L'applicateur peut éventuellement servir d'organe de fermeture à un récipient contenant la composition.

L'applicateur peut encore être agencé pour se fixer de manière amovible sur un dispositif de distribution permettant de prélever la composition.

Le récipient peut encore comporter une extension ayant un logement pouvant recevoir l'applicateur.

L'applicateur et le récipient peuvent, le cas échéant, être contenus dans un dispositif de conditionnement commun, par exemple un coffret, un emballage cartonné, un blister ou un film ou sachet de pelliculage.

L'applicateur peut comporter un organe d'application rotatif agencé pour venir au contact de la région à traiter, voire une pluralité d'organes d'application rotatifs, lesquels peuvent plisser et déplacer la peau lors de leur passage, par exemple.

L'applicateur peut être au moins partiellement magnétique.

L'applicateur peut comporter une lèvre flexible, par exemple agencée à la manière d'une ventouse.

L'applicateur peut comporter un passage permettant à la composition d'être distribuée quand l'applicateur est monté sur le récipient. La composition peut venir au contact de ce passage ou ce dernier peut recevoir un embout de distribution dans lequel circule la composition.

L'applicateur peut ne pas comporter de composés réagissant ensemble selon une réaction exothermique.

L'applicateur peut comporter un vibreur amovible et/ou au moins une électrode amovible reliée à une source électrique, par exemple deux électrodes entre lesquelles existe une différence de potentiel.

Le récipient peut présenter un espace intérieur contenant la composition, de volume variable.

L'applicateur peut présenter une surface d'application définie au moins partiellement par une pièce amovible. Cette dernière est par exemple au moins partiellement composée par un matériau absorbant et comporte par exemple un matériau alvéolaire et/ou des fibres, ou poils, et peut par exemple être détachée de l'applicateur pour être lavée. La pièce amovible peut par exemple améliorer l'étalement de la composition sur la région traitée au moyen de l'applicateur.

L'applicateur peut présenter entre la cavité précitée et la surface d'application une plus petite épaisseur (eₘᵢₙ) inférieure ou égale à 50 mm, mieux inférieure ou égale à 10 mm, encore mieux inférieure ou égale à 1 mm, par exemple comprise entre 0,1 mm et 1 mm, par exemple allant de 0,2 mm à 0,8 mm afin de favoriser le transfert thermique entre la surface d'application et le liquide contenu dans la cavité.

L'applicateur peut comporter au moins un indicateur de température, par exemple par un indicateur thermochromique. L'indicateur de température peut changer de couleur pour signaler à l'utilisateur que la surface d'application est à une température supérieure ou inférieure à un seuil prédéfini, par exemple.

L'applicateur peut comporter un organe de prélèvement de la composition contenue dans le récipient. Cet organe de prélèvement comporte par exemple une mousse, un fritté, un feutre, un tissé, non-tissé, un flocage ou des poils. L'organe de prélèvement peut se situer à une extrémité de l'applicateur opposée à la surface d'application.

L'applicateur peut comporter une première partie assemblée avec au moins une deuxième partie, par exemple par montage à force, encliquetage, vissage, soudage, collage, surmoulage ou sertissage.

La première partie peut définir la surface d'application et peut comporter un matériau relativement dense, par exemple. La deuxième partie peut définir la surface de préhension et peut comporter un matériau moins dense. La première ou la deuxième partie peut comporter une ouverture de remplissage de la cavité contenant un liquide ou autre composé capable d'emmagasiner la chaleur.

Le récipient peut comporter un premier compartiment contenant la composition et un deuxième compartiment pour recevoir l'applicateur et un organe de fermeture permettant de fermer à la fois les premier et deuxième compartiments.

Le récipient peut comporter un logement central pour recevoir au moins partiellement l'applicateur.

L'ensemble de conditionnement et d'application peut comporter un premier applicateur destiné à être refroidi et un deuxième applicateur destiné à être réchauffé. Le premier peut contenir un liquide et comporter un matériau métallique et le deuxième être dépourvu de métal.

La cavité peut être délimitée par une paroi d'épaisseur non constante.

L'applicateur peut être configuré pour que la cavité ne soit pas accessible depuis l'extérieur. Par l'expression « pas accessible depuis l'extérieur », on entend qu'aucun passage pouvant s'ouvrir pour accéder à l'intérieur de la cavité n'est prévu dans l'applicateur, une fois bien entendu que la cavité a été remplie lors de la fabrication de l'applicateur.

L'invention pourra être mieux comprise à la lecture de la description détaillée qui va suivre, d'exemples de mise en oeuvre non limitatifs de celle-ci, et à l'examen du dessin annexé, sur lequel :
- la figure 1 représente en élévation un dispositif réalisé conformément à un exemple de mise en oeuvre de l'invention,
- la figure 2 est une coupe longitudinale, schématique, du dispositif de la figure 1,
- la figure 3 représente isolément une variante de réalisation de l'applicateur,
- la figure 4 est une vue analogue à la figure 1 d'une variante de réalisation du dispositif,
- la figure 5 représente isolément, en élévation, l'applicateur de la figure 4,
- la figure 6 est une vue en perspective d'une variante de réalisation du dispositif,
- la figure 7 est une coupe longitudinale du dispositif de la figure 6,
- les figures 8 à 12 sont des coupes longitudinales, schématiques et partielles, de dispositifs selon des variantes de mise en oeuvre de l'invention,
- les figures 13 et 14 représentent de manière schématique, en perspective, des exemples de kits pour la mise en oeuvre de l'invention,
- la figure 15 illustre l'utilisation du kit de la figure 14,
- la figure 16 représente de manière schématique un autre exemple de kit pour mettre en oeuvre l'invention,
- la figure 17 représente l'applicateur de la figure 16 au moment de l'utilisation,
- les figures 18 à 24, 26 à 28 sont des coupes longitudinales, schématiques, de variantes de réalisation de l'applicateur,
- la figure 25 représente des variantes de réalisation de l'applicateur,
- les figures 29 et 39 représentent de manière schématique, en perspective, d'autres exemples de réalisation de l'applicateur,
- la figure 30 est une coupe longitudinale partielle de l'applicateur de la figure 29,
- les figures 31 à 34 et 38 sont des vues en élévation de variantes de réalisation de l'applicateur,
- les figures 35 à 37 et 40 à 53 sont des coupes longitudinales, partielles et schématiques, de variantes de réalisation de l'applicateur,
- la figure 54 représente, en perspective, un autre exemple de dispositif de conditionnement et d'application,
- la figure 55 est une coupe schématique et partielle de l'applicateur,
- la figure 56 est une vue analogue à la figure 54 d'une variante de réalisation, et
- la figure 57 représente de façon schématique une cellule de sécurité pour l'applicateur.

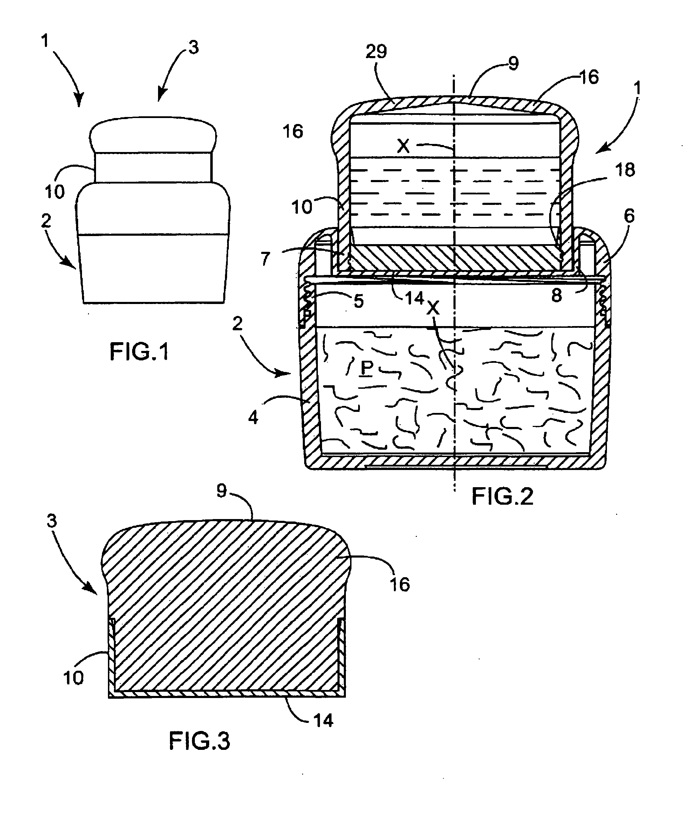

Le dispositif 1 de conditionnement et d'application représenté aux figures 1 et 2 comporte un récipient 2 contenant une composition cosmétique P à appliquer sur le corps et un applicateur 3, d'axe X, pouvant être utilisé lors de l'application de la composition P.

La composition P est par exemple destinée à exercer au moins une action anti-ride, amincissante, hydratante, colorante, anti-acnéique, anti-séboréique, dépigmentante, stimulante, régénérante, apaisante et/ou à dissimuler des défauts cutanés.

La composition cosmétique P est par exemple telle que définie dans la Directive 93/35/CEE du 14 juin 1993 modifiant la Directive 76/768/CEE.

La composition P est par exemple telle qu'elle ne supporte pas un stockage prolongé dans le récipient 2 à une température supérieure ou égale à 55 °C.

La composition P peut contenir, le cas échéant, au moins un composé changeant d'état à une température comprise entre 30 °C et la température à laquelle est porté l'applicateur. Il peut s'agir d'une dispersion de particules d'une cire par exemple.

Dans l'exemple considéré, le récipient 2 se présente sous la forme d'un pot comportant un corps 4 pourvu supérieurement d'un col fileté 5 et d'un couvercle 6 pouvant se fixer de façon amovible sur le col 5, par exemple par vissage.

Le couvercle 6 et le col 5 peuvent comporter des moyens d'étanchéité permettant d'obtenir une fermeture étanche du récipient 2.

Le couvercle 6 comporte une paroi supérieure 8 définissant un logement 7 ouvert vers le haut, dans lequel peut s'engager l'applicateur 3 en l'absence d'utilisation.

L'applicateur 3 est par exemple retenu par friction dans le logement 7.

L'applicateur 3 présente une surface d'application 9 destinée à venir au contact de la région à traiter, par exemple la peau, et une surface de préhension 10 pouvant être saisie par l'utilisateur pour manipuler l'applicateur 3.

La surface d'application 9 est par exemple située du côté opposé au récipient 2 lorsque l'applicateur 3 est reçu dans le logement 7.

La surface de préhension 10 est par exemple une surface latérale s'étendant à partir d'une face inférieure 13 de l'applicateur, par exemple sur plus du tiers de la hauteur totale de l'applicateur, par exemple sur sensiblement la moitié de sa hauteur totale.

Dans l'exemple considéré, l'applicateur 3 comporte une cavité intérieure 12 qui peut contenir un composé L destiné à emmagasiner de la chaleur, par exemple une cire, une huile ou de l'eau. Dans le cas d'une cire par exemple, celle-ci peut avoir un point de fusion compris entre 30 °C et 80 °C par exemple.

La quantité de composé L va par exemple de 0,5 à 10 cm³, en fonction de la chaleur que l'on cherche à emmagasiner.

Dans une variante non illustrée, la cavité 12 est remplie d'une poudre, par exemple du sable.

Dans l'exemple considéré, la cavité intérieure 12 est formée entre une première partie 16 qui définit la surface d'application 9 et une deuxième partie 14 assemblée avec la première.

La première partie 16 est par exemple en verre ou en une céramique compatible avec le passage dans un four à micro-ondes.

La deuxième partie 14 est par exemple en matière plastique et comporte par exemple une lèvre d'étanchéité 18 d'axe X qui vient recouvrir la première partie 16 afin d'obtenir un assemblage étanche.

La première partie 16 comporte une paroi supérieure 29 dont l'épaisseur, mesurée selon l'axe X, peut par exemple décroître en rapprochement de l'axe X de façon à favoriser le transfert thermique entre la cavité 12 et la surface d'application 9 dans la région centrale de l'applicateur 3.

L'assemblage des première et deuxième parties, dans l'exemple de la figure 2, peut se faire de diverses façons, par exemple par vissage ou montage en force, collage, encliquetage, soudure, surmoulage ou sertissage.

Dans la variante illustrée à la figure 3, l'applicateur 3 ne comporte pas la cavité 12 remplie du composé L.

La première partie 16 est alors par exemple pleine et réalisée dans un matériau présentant une capacité thermique et une conductivité thermique suffisantes pour obtenir le résultat recherché. Ce matériau comporte par exemple un verre, une pierre dense ou une matière plastique chargée.

La deuxième partie 14 peut être utile pour isoler thermiquement la surface de préhension 10 et peut être réalisée dans une matière plastique présentant une conductivité thermique inférieure ou égale à 1 Wm⁻¹K⁻¹ ou en bois, par exemple.

L'applicateur 3 peut encore, dans une variante non illustrée, être réalisé d'une seule pièce en un seul matériau.

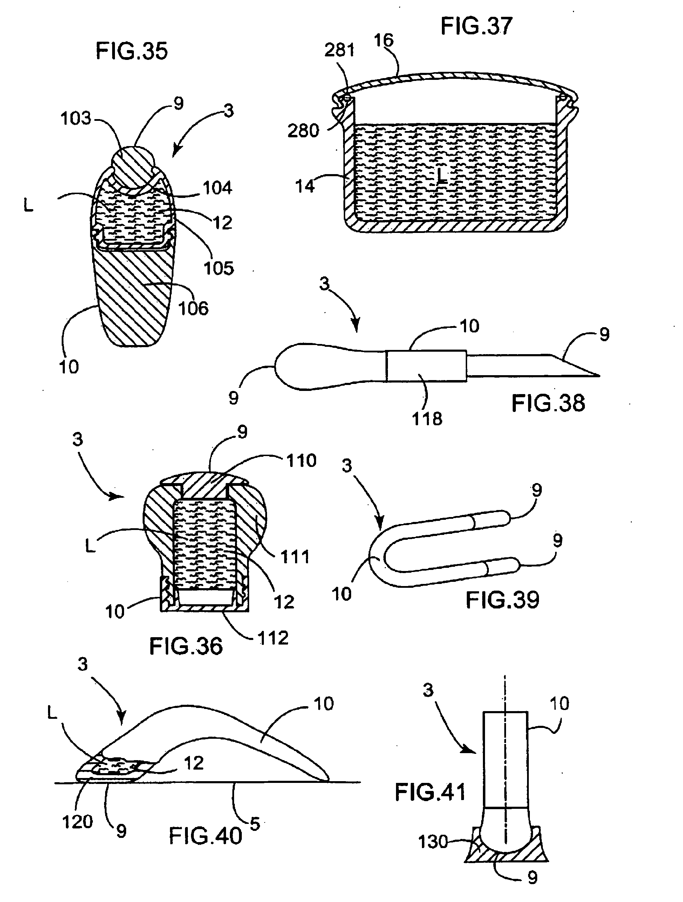

Dans la variante de la figure 37, la première partie 16 est par exemple formée d'une plaque.

La deuxième partie 14 est par exemple agencée pour permettre la fixation de la première partie 16 par encliquetage.

La deuxième partie 14 peut comporter une gorge 180 pouvant accueillir un joint d'étanchéité 281 s'appliquant sur la surface intérieure de la première partie 16. La première partie 16 peut être fixée sur la deuxième partie 14 après remplissage de cette dernière par le composé L.

L'applicateur 3 peut être réalisé avec une forme adaptée à la région à traiter.

Les applicateurs 3 représentés sur les figures 1 à 3 sont relativement larges, étant par exemple plus larges que hauts, et sont destinés par exemple à traiter une région autre que le visage.

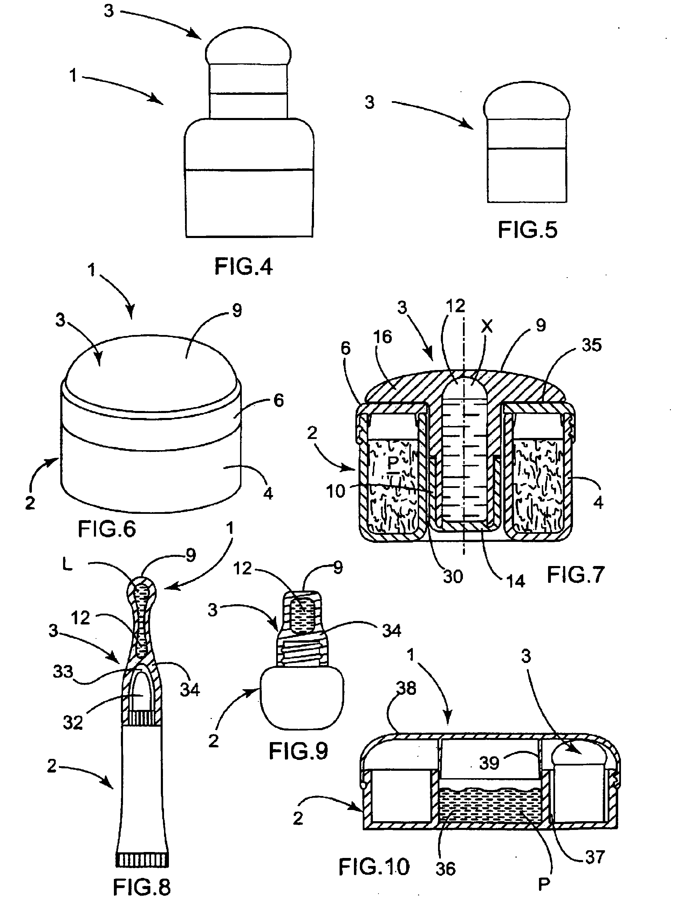

On a représenté à la figure 4 un dispositif de conditionnement et d'application 1 ayant un applicateur 3 plus étroit, mieux adapté au visage. On peut voir sur la figure 5 que cet applicateur 3 est par exemple plus haut que large.

On a représenté aux figures 6 et 7 un dispositif selon une variante de réalisation dans laquelle le récipient 2 présente un logement central 30 pour permettre d'y engager au moins partiellement l'applicateur 3.

Le logement 30 est par exemple traversant, comme illustré.

L'applicateur 3 est par exemple agencé pour reposer par une face d'appui 35 contre le dessus du couvercle du récipient 2, l'applicateur 3 présentant par exemple une forme générale de champignon. La face d'appui 35 peut ainsi présenter une forme annulaire.

L'applicateur 3 de la figure 7 peut être réalisé comme illustré avec la cavité 12 et celle-ci s'étend par exemple au moins partiellement dans le logement 30 lorsque l'applicateur 3 est en place sur le récipient 2.

La face d'appui 35 peut être définie par la première partie 16.

Pour utiliser les applicateurs 3 des exemples des figures 1 à 5, l'utilisateur peut les réchauffer en les plaçant dans un four à micro-ondes.

Le récipient 2 contenant la composition cosmétique P peut être maintenu à une température inférieure à celle de l'applicateur 3, par exemple en étant conservé à température ambiante hors du four à micro-ondes.

Ensuite, l'utilisateur peut prélever la composition P dans le récipient 2, par exemple avec le doigt, et l'appliquer sur la région à traiter ou sur l'applicateur 3.

Ensuite, l'applicateur 3 peut être amené au contact de la région à traiter.

L'applicateur 3 peut être utilisé en étant déplacé au contact de la peau, par exemple par des mouvements circulaires ou rectilignes, pour exercer, par exemple, une action de massage et/ou, le cas échéant, étaler la composition P. L'utilisateur peut encore procéder par pressions successives, sans mouvement sensible de l'applicateur sur la peau, pour effectuer une thermopuncture par exemple. L'applicateur 3 peut effleurer la peau. On pourra effectuer une sorte de repassage à chaud de la peau ou d'un substrat sur la peau.

L'applicateur 3 peut être utilisé, le cas échéant, pour prélever la composition P dans le récipient 2.

On a illustré à la figure 8 la possibilité pour le récipient 2 de se présenter sous la forme d'un tube ayant un embout de distribution obturé par un capuchon de fermeture 32.

L'applicateur 3 peut être agencé pour se fixer sur le capuchon de fermeture 32 grâce à un évidement 33 réalisé dans le corps 34 de l'applicateur.

Le maintien de l'applicateur 3 sur le capuchon 32 peut être assuré par exemple par friction ou autrement, par exemple par encliquetage ou vissage.

Le corps 34 peut être réalisé avec la cavité 12 recevant le composé L, comme illustré, ou être plein.

Pour utiliser l'applicateur 3, l'utilisateur peut le séparer du récipient 2 et le placer au four à micro-ondes.

Ensuite, l'utilisateur peut distribuer la composition contenue dans le récipient 2 sur l'applicateur 3 ou sur la région à traiter.

L'utilisateur peut se servir du corps 34 comme d'un organe de préhension sans replacer l'applicateur 3 sur celui-ci ou en variante utiliser l'applicateur 3 après l'avoir replacé sur le récipient 2, ce dernier définissant alors la surface de préhension.

On a représenté à la figure 9 une variante de réalisation dans laquelle le récipient 2 est un flacon pourvu d'un col et le corps 34 sert de bouchon de fermeture étant vissé sur le col.

La composition P peut être contenue dans un récipient 2 tel que celui illustré à la figure 10, qui comporte un premier compartiment 36 pour recevoir la composition P et un deuxième compartiment 37 pour recevoir l'applicateur 3.

Ces deux compartiments 36 et 37 sont dans l'exemple considéré obturés par un couvercle commun 38, lequel comporte par exemple une jupe d'étanchéité 39 afin de fermer de façon étanche le premier compartiment 36.

Dans l'exemple de la figure 10, le couvercle 38 est agencé pour se visser mais dans des variantes non illustrées, le couvercle 38 se fixe autrement, étant par exemple emboîté ou retenu par une articulation sur le récipient 2.

Le deuxième compartiment 37 peut, le cas échéant, contenir plusieurs applicateurs 3 ayant des formes différentes et/ou destinés à être utilisés différemment, par exemple en étant refroidi pour au moins l'un d'entre eux et réchauffé pour au moins un autre d'entre eux.

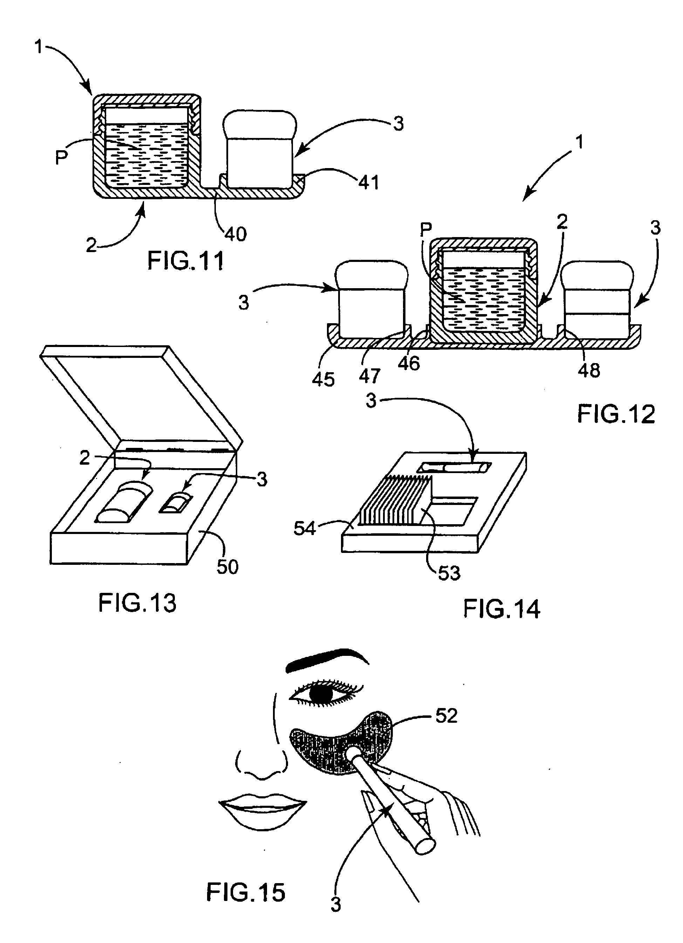

La composition P peut, comme illustré à la figure 11, être contenue dans un récipient 2 comportant une extension 40 agencée pour accueillir l'applicateur 3.

L'extension 40 s'étend par exemple latéralement et peut comporter un logement 41 de forme adaptée à recevoir l'applicateur 3.

La composition P à appliquer peut être proposée à l'utilisateur associée à au moins deux applicateurs 3, l'un étant par exemple destiné à être refroidi et l'autre à être réchauffé.

Ces deux applicateurs 3 peuvent être proposés à l'utilisateur sous la forme d'un kit avec le récipient 2 contenant la composition P.

Le kit comporte, par exemple, un support 45 pouvant accueillir le récipient 2 et les applicateurs 3 en l'absence d'utilisation, comme illustré à la figure 12.

Le support 45 peut comporter par exemple un logement 46 pour recevoir le récipient 2 contenant la composition P et deux logements 47 et 48 pour recevoir chacun un applicateur 3.

Le récipient 2 est réalisé d'une seule pièce avec le support 45 dans une variante non illustrée.

Le récipient 2 peut également être proposé à l'utilisateur avec au moins un applicateur 3 dans un emballage tel que par exemple un coffret 50, comme illustré à la figure 13.

La composition P peut se présenter sous la forme d'une poudre, crème, pâte, gel ou liquide, ou encore imprégner et/ou recouvrir un substrat tel que par exemple un tissé, un non-tissé, une mousse ou un feutre.

Ce substrat 52, sous la forme d'un masque ou d'un patch par exemple, peut être appliqué sur la peau, puis l'applicateur 3 réchauffé être amené à son contact, comme illustré à la figure 15.

Le substrat 52 peut être contenu dans un emballage individuel 53 et être proposé à l'utilisateur sous la forme d'un kit en association avec au moins un applicateur 3, l'ensemble étant par exemple contenu dans un emballage 54, comme illustré à la figure 14.

Une pluralité de substrats peut encore être proposée à l'utilisateur, non pas dans des emballages individuels mais dans un emballage commun, dans une variante non illustrée.

Le substrat imprégné de composition peut éventuellement être sensiblement anhydre et peut avoir à être mouillé par un solvant tel que de l'eau par exemple, au moment de l'utilisation.

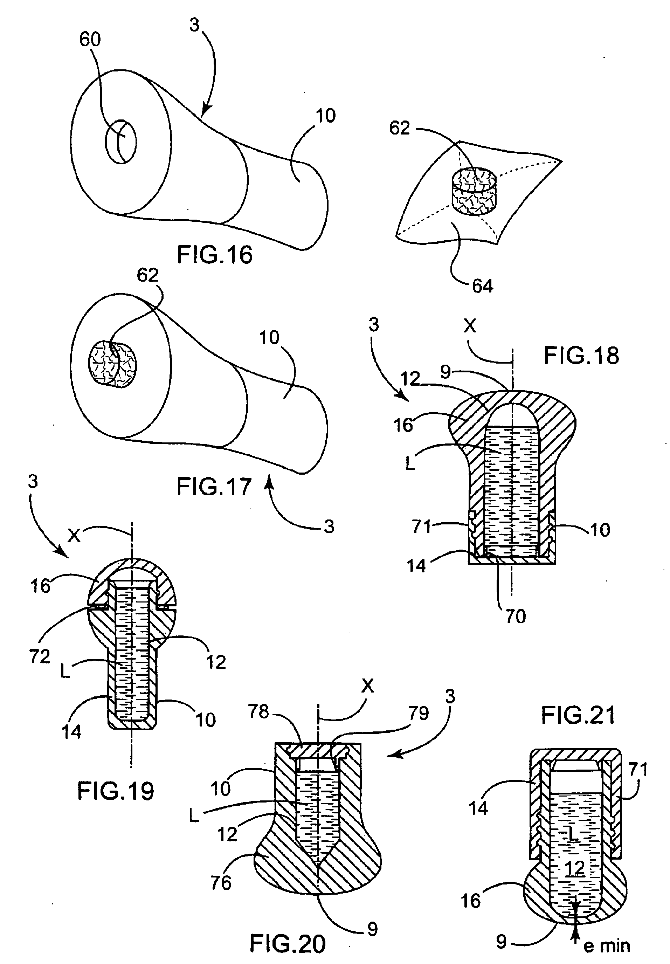

On a représenté à la figure 16 un autre exemple de kit pour la mise en oeuvre de l'invention.

L'applicateur 3 comporte un logement 60 destiné à recevoir un substrat 62 contenant la composition à appliquer, voire la composition elle-même sous la forme d'un bloc de forme adaptée à être reçue dans le logement 60.

Le substrat 62 ou le bloc de composition peut être contenu dans un emballage individuel 64, par exemple.

La quantité de composition contenue dans le substrat 62 ou constituant le bloc peut correspondre à un usage unique.

Lors de l'utilisation, l'utilisateur extrait le substrat 62 ou le bloc de composition de l'emballage 64 et l'introduit dans le logement 60 de l'applicateur.

La profondeur du logement 60 est par exemple inférieure à celle du substrat 62 de façon à ce que l'utilisateur puisse appliquer la composition sur la région à traiter.

Le logement 60 est par exemple réalisé dans une partie en verre, céramique ou pierre de l'applicateur 3, afin de favoriser l'échange de chaleur entre le substrat 62 ou le bloc de composition et l'applicateur 3.

La mise en place du substrat 62 ou du bloc de composition peut s'effectuer sur l'applicateur réchauffé.

Après l'application, le substrat 62 peut être retiré du logement 60.

L'applicateur 3 peut être réalisé de multiples autres façons encore.

La cavité 12 qui contient le composé L peut notamment être fermée de diverses manières.

On a représenté à la figure 18 un exemple de réalisation dans lequel les première 16 et deuxième 14 parties coopèrent par vissage et la deuxième partie 14 comporte une lèvre d'étanchéité 70 qui vient s'appliquer sur une surface radialement intérieure de la première partie afin de fermer de façon étanche la cavité 12.

La deuxième partie 14 peut comporter une jupe filetée 71 qui recouvre partiellement la première partie 16 et qui définit la surface de préhension 10.

Dans la variante de réalisation de la figure 19, les première 16 et deuxième 14 parties sont assemblées avec interposition d'un joint d'étanchéité 72.

La deuxième partie 14 est par exemple filetée du côté de la première partie 16, laquelle peut être vissée sur la deuxième partie 14.

L'applicateur 3 peut encore comporter un corps 76 définissant les surfaces de préhension 10 et d'application 9, comme illustré à la figure 20. Ce corps peut être évidé pour définir la cavité 12 remplie du composé L.

La cavité 12 peut être fermée par exemple au moyen d'un bouchon 78 qui peut être fixé de diverses manières sur le corps 76, étant par exemple encliqueté sur celui-ci. Le bouchon 78 peut comporter une lèvre annulaire d'étanchéité 79 s'appliquant sur une surface du corps 76 délimitant la cavité 12.

On a représenté à la figure 21 une variante de réalisation assez similaire à celle de la figure 18.

Toutefois, la deuxième partie 14 comporte une jupe 71 qui s'étend jusqu'à la portion renflée de la première partie.

En outre, l'épaisseur de la première partie 16 entre la cavité 12 et la surface d'application est plus faible.

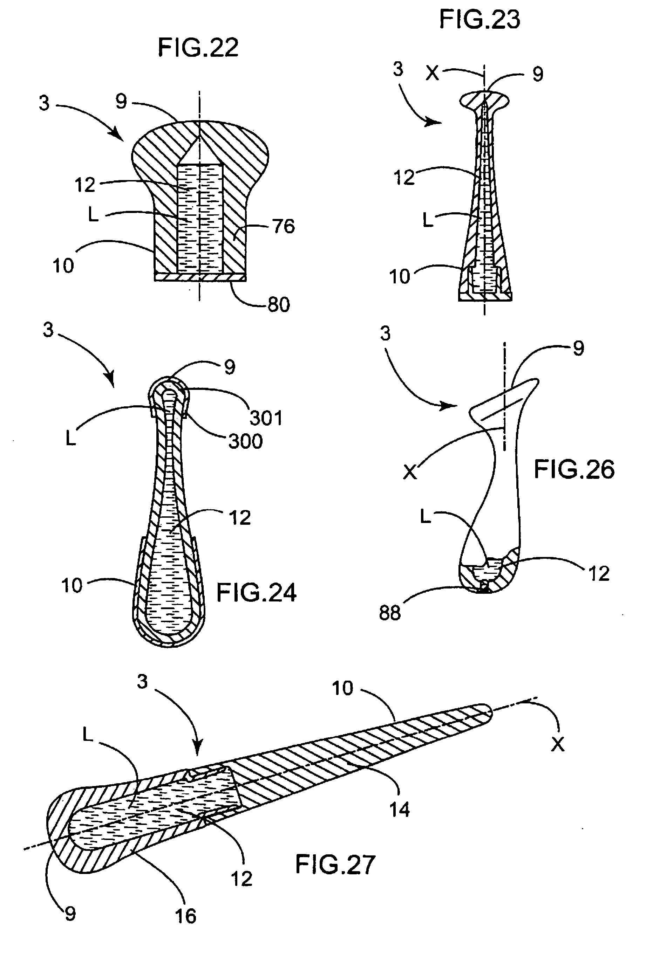

L'applicateur 3 représenté à la figure 22 diffère de celui illustré à la figure 20 par le fait que la cavité 12 est fermée par un fond 80 qui est par exemple soudé sur le corps 76 autour de l'ouverture servant au remplissage de la cavité 12.

Le fond 80 est par exemple une feuille d'un matériau imperméable au composé L, qui peut être transparent le cas échéant.

L'applicateur 3 peut être réalisé avec des formes extérieures très différentes, par exemple une forme allongée avec une tête élargie, comme illustré à la figure 23.

La cavité 12 peut s'étendre sur une hauteur plus ou moins importante de l'applicateur.

Dans l'exemple de la figure 23, la cavité 12 s'étend sur plus de la moitié de la longueur de l'applicateur 3, en l'espèce pratiquement sur toute sa longueur.

Dans l'exemple de réalisation de la figure 24, l'applicateur 3 présente une tête arrondie.

On a illustré sur cette figure la possibilité pour l'applicateur de comporter une surface d'application 9 définie par un revêtement 300 recouvrant une paroi 301 délimitant la cavité 12. Le revêtement 300 est par exemple une membrane en élastomère, un tissé ou un non-tissé.

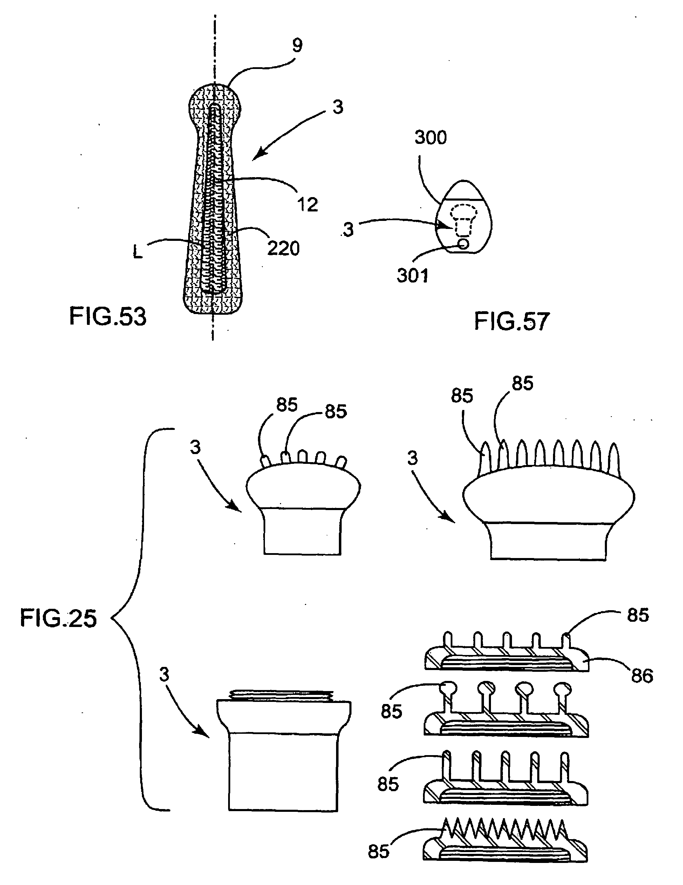

La surface d'application 9 de l'applicateur 3 peut comporter des reliefs, par exemple des picots 85 comme illustré à la figure 25. Les reliefs 85 peuvent être réalisés dans un matériau élastomère, par exemple. Le cas échéant, les reliefs 85 peuvent être réalisés sur une pièce amovible 86, ce qui permet à l'utilisateur de choisir les reliefs 85 en fonction du traitement à effectuer. La fixation de la pièce 86 peut s'effectuer par vissage, par exemple.

L'applicateur 3 peut comporter une surface d'application 9 ayant une portion plane comme illustré à la figure 26, laquelle peut s'étendre obliquement par rapport à l'axe longitudinal X de l'applicateur 3, par exemple.

On peut voir également sur la figure 26 que la cavité 12 peut comporter une ouverture pour son remplissage avec le composé L, ouverture qui peut être obturée par un bouchon 88 formé par exemple par une goutte de colle.

On a représenté à la figure 27 un applicateur 3 qui comporte une première partie 16 définissant la cavité 12 remplie du composé L et une deuxième partie 14 se présentant sous la forme d'un manche allongé qui peut ne pas être parcouru par la cavité 12, comme illustré.

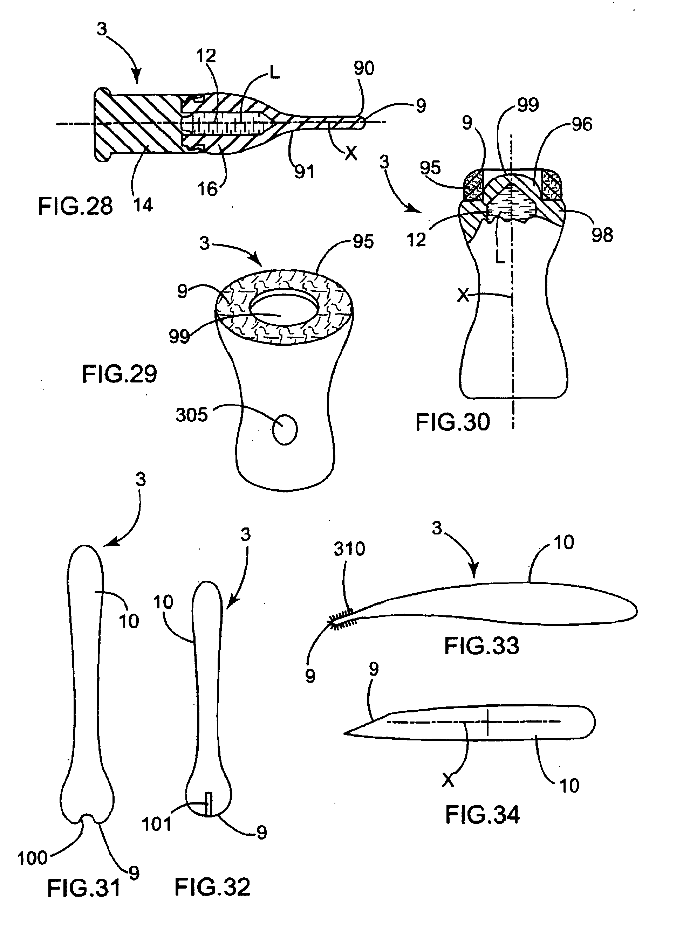

La surface d'application peut encore être définie par une tête 90 qui se raccorde au reste de l'applicateur par une portion étroite 91, comme illustré à la figure 28. La portion étroite 91 peut être flexible le cas échéant, et fléchir lors de l'utilisation.

La première partie 16 est par exemple encliquetée dans la deuxième partie 14, la cavité 12 étant formée à l'intérieur de la première partie 16 et obturée à une extrémité par la deuxième partie 14.

La surface d'application 9 de l'applicateur 3 peut être définie au moins partiellement par une pièce rapportée 95 réalisée par exemple dans un matériau élastiquement déformable, par exemple une mousse, comme représenté aux figures 29 et 30.

La pièce rapportée 95 peut présenter une forme annulaire et se monter sur une extension 96 du corps 98 de l'applicateur. Le sommet 99 de l'extension 96 peut également servir, le cas échéant, de surface d'application. La pièce 95 peut être nettoyée après avoir été utilisée.

On a illustré à la figure 29 la possibilité pour l'applicateur 3 de comporter un indicateur de température 305, lequel peut changer de couleur et signaler à l'utilisateur que la surface d'application 9 est à une température acceptable pour le traitement à effectuer.

La surface d'application 9 peut comporter, comme on le voit sur la figure 31, au moins un renfoncement 100 permettant une accumulation de produit, voire une fente 101 comme illustré à la figure 32.

La surface d'application 9 peut être définie à une extrémité par une pointe, comme illustré à la figure 33, voire par une face d'extrémité biseautée comme illustré à la figure 34.

On a également illustré à la figure 33 la possibilité pour la surface d'application 9 de comporter un flocage 310.

La surface d'application 9 peut encore être définie, comme illustré à la figure 35, par une masse 103 d'un matériau conducteur de la chaleur, fixée dans un logement 104 d'une première partie 105 de l'applicateur 3. Cette première partie peut définir la cavité 12 comportant le composé L, et peut être supportée par une partie de préhension 106 réalisée dans un matériau moins bon conducteur de la chaleur que celui de la première partie 105. La masse 103 est par exemple une céramique, un verre ou une pierre dense.

On a illustré à la figure 36 la possibilité pour la surface d'application 9 d'être formée par une pièce 110 qui est en contact direct avec le composé L contenu dans la cavité 12.

La pièce 110 est par exemple en verre et la cavité 12 est par exemple formée à l'intérieur d'un corps 111 en matière plastique isolante.

Un bouchon 112 peut être vissé sur le corps 111 pour fermer inférieurement la cavité 12.

L'applicateur 3 peut comporter deux surfaces d'application 9 ayant des formes différentes, comme illustré à la figure 38, par exemple une surface d'application ayant une forme arrondie et une surface d'application ayant une forme biseautée.

Une bague isolante 118 peut être prévue dans une région médiane de l'applicateur 3 pour définir la surface de préhension 10.

Les surfaces d'application 9 présentant des formes différentes sont par exemple situées à des extrémités opposées de l'applicateur 3.

Ce dernier peut encore comporter des surfaces d'application 9 ayant des formes différentes ou non, situées d'un même côté, comme illustré à la figure 39.

L'applicateur 3 peut par exemple présenter une forme de U, les extrémités du U définissant les surfaces d'application 9 et la base du U la surface de préhension 10.

L'applicateur 3 peut être agencé de manière à permettre, lorsqu'il repose sur une surface plane S, comme illustré à la figure 40, au composé L contenu dans la cavité 12 de venir au contact de la paroi 120 qui définit la surface d'application 9.

Le cas échéant, l'applicateur 3 peut être placé sur un support 130 lorsqu'il n'est pas utilisé, comme illustré à la figure 41.

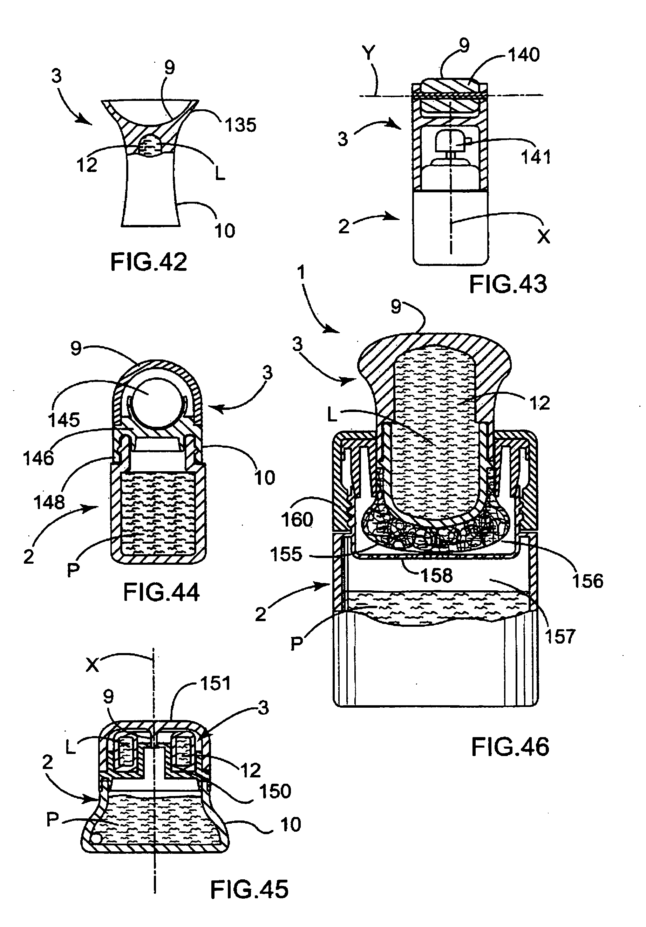

L'applicateur 3 peut être au moins partiellement élastiquement déformable et la surface d'application 9 peut être définie au moins partiellement par une lèvre flexible 135 permettant de créer un effet de ventouse, comme illustré à la figure 42.

La surface d'application 9 peut également être définie par un organe mobile, notamment rotatif, par exemple un rouleau 140 comme illustré à la figure 43, tournant autour d'un axe de rotation Y qui est par exemple perpendiculaire à l'axe longitudinal X.

On a illustré sur cette figure la possibilité pour le récipient 2 d'être équipé d'un dispositif de distribution 141 tel que par exemple une pompe ou une valve, permettant de distribuer la composition sur la région à traiter ou sur la surface d'application 9 préalablement à sa mise en contact avec la peau.

La surface d'application 9 peut encore être définie par un organe rotatif tel qu'une bille 145, comme illustré à la figure 44.

Cette bille peut être portée par un support 146 qui peut être fixé de manière amovible sur le récipient 2. Ainsi, l'utilisateur peut détacher le support 146 afin de le réchauffer sans exposer au micro-ondes la composition P contenue dans le récipient. La bille peut, le cas échéant, comporter la cavité 12 et le composé L.

Le support 146 peut être remis en place sur le récipient 2 une fois réchauffé et le récipient 2 être utilisé comme organe de préhension.

En variante, c'est une jupe 148 de l'applicateur 3, qui permet sa fixation sur le récipient 2, qui peut définir la surface de préhension 10.

On a représenté à la figure 45 une variante de réalisation dans laquelle la surface d'application 9 peut être définie par un applicateur 3 de forme générale annulaire, pouvant être placé autour d'une cheminée 150 du récipient 2 par laquelle la composition P est distribuée.

Un organe de fermeture 151 peut être mis en place sur le récipient 2 de façon à obturer la cheminée 150 en l'absence d'utilisation. Pour utiliser l'applicateur 3, l'utilisateur enlève celui-ci du récipient 2 et le place dans un four à micro-ondes.

Ensuite, une fois réchauffé, l'applicateur 3 est remis en place autour de la cheminée 150 et le récipient 2 peut être utilisé comme organe de préhension pour amener la surface d'application 9 au contact de la région à traiter.

Dans l'exemple de la figure 46, l'applicateur 3 comporte une surface d'application 9 destinée à apporter de la chaleur dans la région à traiter et un organe 155 de prélèvement du produit contenu dans le récipient 2.

Cet organe de prélèvement 155 est par exemple situé sur l'applicateur 3 du côté opposé à la surface d'application 9.

Le récipient 2 comporte par exemple un logement 156 pour recevoir l'organe de prélèvement 155, ce logement 156 étant séparé d'un espace 157 contenant la composition P par une paroi ajourée 158 qui limite la quantité dont peut se charger l'organe de prélèvement 155. Ce dernier peut servir à appliquer la composition P sur la région à traiter, le cas échéant.

L'applicateur 3 peut être solidaire d'un organe de fermeture 160 du récipient 2, par exemple agencé pour se visser sur celui-ci.

Pour utiliser le dispositif 1 de la figure 46, l'utilisateur sépare l'applicateur 3 du récipient et place celui-ci dans un four à micro-ondes. Ensuite, l'utilisateur prélève la composition P dans le logement 156, par exemple après avoir secoué le dispositif 1 avec l'applicateur 3 en place, et peut appliquer la composition contenue dans et/ou sur l'organe de prélèvement 155 au moyen de ce dernier. Une fois la composition appliquée, l'utilisateur peut retourner l'applicateur 3 et utiliser la surface d'application 9 pour apporter de la chaleur. L'utilisateur sépare l'applicateur 3 du récipient et place celui-ci dans un four à micro-ondes.

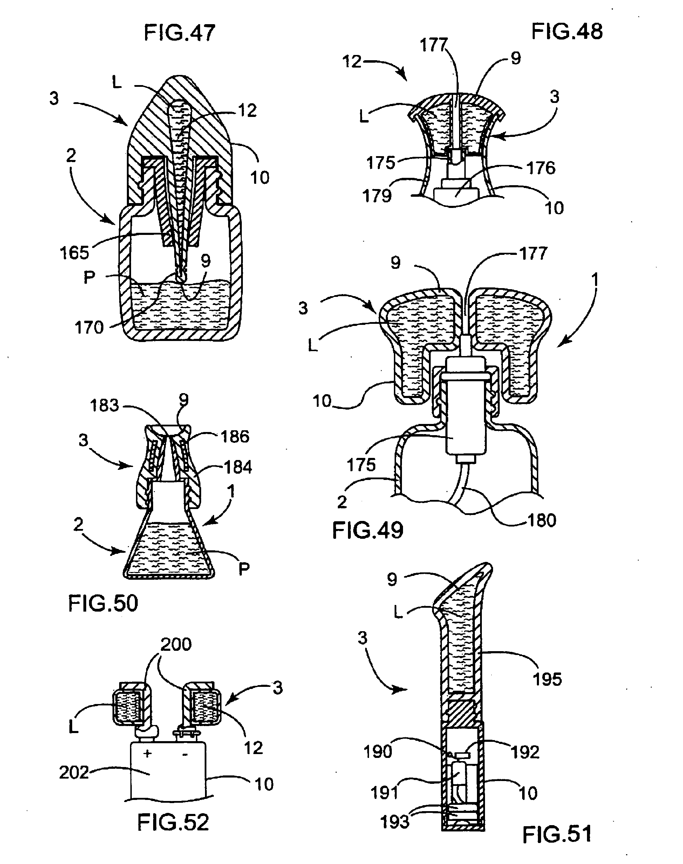

Dans l'exemple de la figure 47, la surface d'application 9 est agencée pour venir au contact de la composition à l'intérieur du récipient 2. Ce dernier peut comporter un organe d'essorage 165 qui permet de retirer un excès de produit éventuellement présent sur l'applicateur 3.

L'applicateur 3 peut comporter des reliefs 170 permettant d'accroître la quantité de produit dont se charge l'applicateur et/ou permettant de conférer plus de souplesse à l'applicateur lors de son passage sur la région à traiter.

L'applicateur 3 peut être agencé pour se monter sur un dispositif de distribution 175 tel qu'une pompe ou valve, comportant une tige 176 dont l'enfoncement et/ou l'inclinaison provoque la distribution de la composition.

L'applicateur 3 peut comporter un canal intérieur 177 permettant au produit délivré par la tige 176 de gagner la surface d'application 9.

Dans l'exemple de la figure 48, l'applicateur 3 comporte une jupe d'habillage 179 permettant de dissimuler tout ou partie du dispositif de distribution 175 et définissant également une surface de préhension pour l'utilisateur.

Le dispositif 1 de la figure 48 s'utilise en séparant dans un premier temps l'applicateur 3 du dispositif de distribution 175 et en réchauffant l'applicateur 3 indépendamment du dispositif de distribution 175.

Ensuite, l'applicateur 3 est remis en place sur le dispositif de distribution 175 et l'utilisateur peut, en actionnant l'applicateur 3, provoquer la distribution de produit au travers du canal 177.

La figure 49 représente une variante dans laquelle le dispositif de distribution 175 comporte une pompe alimentée par un tube plongeur 180.

Dans l'exemple de la figure 50, l'applicateur 3 peut se visser sur le col d'un récipient et présente par exemple une surface d'application 9 convexe vers l'extérieur et dans le fond de laquelle débouche un orifice 183 d'amenée de la composition P. Le corps 184 de l'applicateur peut comporter un insert 186 destiné à accroître l'inertie thermique de l'applicateur 3.

Quelle que soit la forme de l'applicateur 3, celui-ci peut être équipé d'un vibreur 190, comme illustré à la figure 51.

Un tel vibreur 190 comporte par exemple un moteur 191 entraînant en rotation une masse excentrée 192 de façon à provoquer des vibrations. Le moteur 191 peut être alimenté par une ou plusieurs piles 193, éventuellement rechargeables.

Le vibreur 190 est agencé pour se fixer de manière amovible sur une partie 195 de l'applicateur comportant la surface d'application 9, de façon à permettre à l'utilisateur d'utiliser le même vibreur avec des surfaces d'application 9 différentes et de pouvoir placer l'applicateur sans le vibreur dans un four à micro-ondes.

L'applicateur 3 peut également comporter au moins une électrode amovible, par exemple deux électrodes 200 afin de stimuler électriquement la région traitée lors de l'application. Ces électrodes sont par exemple reliées électriquement à une pile 202 qui définit la surface de préhension 10.

L'applicateur 3 peut permettre de réchauffer les électrodes 200.

Pour utiliser l'applicateur 3 de la figure 52, l'utilisateur peut séparer l'applicateur 3 de la pile 202 et des électrodes et le placer dans le four à micro-ondes.

Une fois la température souhaitée atteinte, l'utilisateur peut replacer l'applicateur 3 sur la pile 202 et se servir de cette dernière comme d'un organe de préhension pour amener les électrodes 200 au contact de la région à traiter.

L'applicateur 3 peut également présenter des propriétés magnétiques.

A titre d'exemple, on a représenté à la figure 53 un applicateur 3 qui comporte un corps 220 comportant une charge de particules magnétiques.

Ce corps 220 peut également définir comme dans l'exemple illustré au moins partiellement la cavité 12 contenant le composé L. La présence d'un champ magnétique peut accroître, le cas échéant, la capacité thermique de l'applicateur 3.

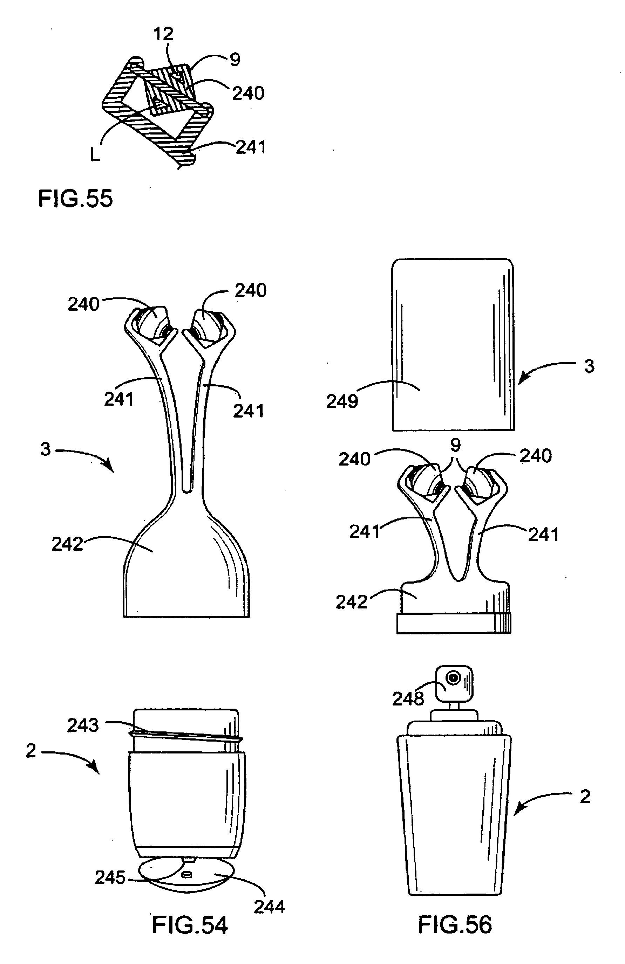

On a représenté aux figures 54 et 55 un dispositif de conditionnement et d'application ayant un applicateur 3 comportant deux rouleaux 240 montés sur des tiges flexibles 241 reliées à un socle 242 permettant de rendre l'applicateur 3 solidaire du récipient 2.

Ce dernier comporte par exemple à une extrémité un filetage 243 pour la fixation de l'applicateur 3 sur le récipient 2 et à l'extrémité opposée un couvercle 244 permettant d'obturer un orifice de distribution 245.

Les rouleaux 240 sont agencés pour présenter la capacité thermique recherchée et peuvent présenter des cavités remplies du composé L.

Dans la variante illustrée à la figure 56, le récipient 2 est équipé d'un dispositif de distribution 248 tel qu'une pompe ou valve. La composition est par exemple contenue sous pression dans le récipient 2.

L'applicateur 3 peut comporter, comme illustré, un capot de protection 249 protégeant la surface d'application 9 en l'absence d'utilisation.

Le cas échéant, comme illustré à la figure 57, l'applicateur 3 peut être placé dans une cellule de sécurité 300 lorsqu'il doit être réchauffé au moyen d'un four à micro-ondes.

Cette cellule de sécurité 300 peut comporter par exemple un indicateur de température 301.

L'invention n'est pas limitée aux exemples qui viennent d'être décrits.

On peut notamment combiner entre elles les particularités de réalisation des divers exemples illustrés.

On peut par exemple équiper d'un vibreur l'un quelconque des applicateurs décrits.

L'expression « comportant un » doit être comprise comme étant synonyme de « comportant au moins un », sauf si le contraire est spécifié.

## Revendications

1. Ensemble comportant :
- une composition cosmétique ou dermatologique contenue dans un récipient ou substrat,
- un applicateur (3) pouvant être séparé du récipient ou substrat et ayant une surface d'application (9), qui n'est pas rotative, pour traiter une région de peau, comportant au moins un matériau ayant un comportement thermique tel que lorsqu'il est réchauffé à une température de 50 °C ne causant pas de lésion thermique à la peau lors du contact de la surface d'application avec la peau pendant 15 s, la surface d'application conserve pendant au moins 30 s après cette application une température supérieure ou égale à 30 °C,
l'applicateur comportant au moins une cavité (12) contenant un composé liquide (L) pouvant être réchauffé à une température supérieure ou égale à 30 °C et inférieure ou égale à 80 °C, la cavité étant fermée.

2. Ensemble selon la revendication 1, l'applicateur (3) étant destiné à être réchauffé dans un four à micro-ondes.

3. Ensemble selon l'une quelconque des revendications précédentes, le composé (L) changeant d'état lorsque réchauffé à une température comprise entre 30 °C et 80 °C.

4. Ensemble selon l'une quelconque des revendications précédentes, comportant au moins 0,2 cm³ dudit composé, mieux entre 1 et 80 cm³ du composé.

5. Ensemble selon l'une quelconque des revendications précédentes, l'applicateur comportant un matériau non métallique de densité supérieure ou égale à 1,5 g/cm³, définissant au moins partiellement la surface d'application (9) destinée à venir au contact de la région à traiter.

6. Ensemble selon la revendication 5, le matériau étant une pierre ou du verre.

7. Ensemble selon l'une quelconque des revendications précédentes, l'applicateur comportant un matériau définissant au moins partiellement une surface d'application (9) destinée à venir au contact de la région à traiter et présentant une conductivité thermique supérieure ou égale à 1 Wm⁻¹ K⁻¹, mieux 40 Wm⁻¹ K⁻¹.

8. Ensemble selon l'une quelconque des revendications 1 à 7, l'applicateur comportant un matériau de capacité calorifique massique supérieure ou égale à 500 J kg⁻¹ K⁻¹, mieux 1 000 J kg⁻¹ K⁻¹, encore mieux 2 000 J kg⁻¹ K⁻¹.

9. Ensemble selon l'une quelconque des revendications précédentes, la surface d'application (9) étant définie au moins partiellement par un matériau présentant une inertie thermique supérieure ou égale à 1 000 Jm⁻² K⁻¹ s^{-1/2}, mieux supérieure ou égale à 5 000, encore mieux 10 000 Jm⁻² K⁻¹ s^{-1/2}.

10. Ensemble selon l'une quelconque des revendications 1 à 9, l'applicateur présentant une masse supérieure ou égale à 15 g.

11. Ensemble selon l'une quelconque des revendications 1 à 10, l'applicateur présentant une surface de préhension définie (10) au moins partiellement par un matériau présentant une conductivité thermique inférieure ou égale à 1 Wm⁻¹K⁻¹, mieux inférieure ou égale à 0,5 Wm⁻¹K⁻¹, voire à 0,1 Wm⁻¹K⁻¹.

12. Ensemble selon l'une quelconque des revendications 1 à 11, l'applicateur étant agencé pour se fixer de manière amovible sur le récipient.

13. Ensemble selon l'une quelconque des revendications 1 à 11, le substrat étant agencé pour se fixer de manière amovible sur l'applicateur.

14. Ensemble selon l'une quelconque des revendications 1 à 12, l'applicateur et le récipient étant contenus dans un dispositif de conditionnement commun.

15. Ensemble selon l'une quelconque des revendications 1 à 14, l'applicateur étant au moins partiellement magnétique.

16. Ensemble selon l'une quelconque des revendications 1 à 15, l'applicateur comportant une lèvre flexible destinée à venir au contact de la région à traiter.

17. Ensemble selon la revendication 12, l'applicateur comportant un passage permettant à la composition d'être distribuée quand l'applicateur est monté sur le récipient.

18. Ensemble selon l'une quelconque des revendications précédentes, l'applicateur comportant un vibreur amovible.

19. Ensemble selon l'une quelconque des revendications précédentes, l'applicateur comportant une électrode amovible reliée à une source électrique.

20. Ensemble selon l'une quelconque des revendications précédentes, l'applicateur présentant une surface d'application définie au moins partiellement par une pièce amovible (95), notamment au moins partiellement en un matériau absorbant.

21. Ensemble selon la revendication 1, l'applicateur comportant entre la cavité (12) et une surface d'application (9) à amener au contact de la région à traiter une paroi ayant une plus petite épaisseur (eₘᵢₙ) inférieure ou égale à 50 mm, notamment comprise entre 0,1 mm et 50 mm, mieux inférieure ou égale à 1 mm, encore mieux à 0,5 mm.

22. Ensemble selon l'une quelconque des revendications précédentes, l'applicateur étant agencé pour, lorsque réchauffé à 50 °C et appliqué sur la peau, conserver pendant 1 mn, mieux 15 mn ou 30 mn, une température supérieure ou égale à 30 °C, notamment au niveau de la surface d'application.

23. Ensemble selon l'une quelconque des revendications précédentes, l'applicateur ne comportant pas de composés réagissant ensemble selon une réaction exothermique.

24. Ensemble selon l'une quelconque des revendications précédentes, l'applicateur étant dépourvu de source électrique ou de moyen de raccordement à une source électrique.

25. Ensemble selon l'une quelconque des revendications précédentes, comportant une cellule de sécurité agencée pour recevoir l'applicateur lorsque celui-ci est placé dans un four à micro-ondes.

26. Ensemble selon l'une quelconque des revendications précédentes, l'applicateur comportant un indicateur thermochromique.

27. Ensemble selon l'une quelconque des revendications précédentes, la cavité étant délimitée par une paroi d'épaisseur non constante.

28. Ensemble selon la revendication 11, les surfaces d'application (9) et de préhension (10) étant définies respectivement par deux parties assemblées l'une sur l'autre.

29. Procédé de traitement cosmétique, non thérapeutique, d'au moins une région du corps humain, comportant les étapes suivantes :
a) réchauffer à une température supérieure ou égale à 30 °C un applicateur (3) d'un ensemble tel que défini dans l'une quelconque des revendications précédentes, puis
b) charger l'applicateur ainsi réchauffé avec une composition cosmétique (P) à une température plus proche de la température ambiante que celle de l'applicateur et ayant avec cette dernière une différence d'au moins 5 °C, et
appliquer la composition en utilisant l'applicateur, ou
c) avant ou après l'étape a), appliquer sur la région à traiter au moins une composition cosmétique et après l'étape a) amener l'applicateur (3) ainsi réchauffé au contact de la région à traiter.

30. Procédé selon la revendication 29, comportant l'application sur la région à traiter d'un substrat (52) portant la composition, l'applicateur étant amené au contact du substrat ainsi appliqué.

31. Procédé selon l'une des revendications 29 ou 30, l'applicateur étant réchauffé en étant introduit dans un four à micro-ondes.

## Claims

1. Assembly comprising:
- a cosmetic or dermatological composition contained in a container or substrate,
- an applicator (3) that can be separated from the container or substrate and has an applicator surface (9), that does not rotate, for treating a region of skin, comprising at least one material having a thermal behaviour such that when it is heated up to a temperature of 50°C, with no thermal lesions being brought about on the skin when the applicator surface is in contact with the skin for 15 s, the applicator surface retains a temperature greater than or equal to 30°C for at least 30 s after this application,
the applicator comprising at least one cavity (12) containing a liquid compound (L) that can be heated up to a temperature greater than or equal to 30°C and less than or equal to 80°C, the cavity being closed.

2. Assembly according to Claim 1, the applicator (3) being intended to be heated up in a microwave oven.

3. Assembly according to either one of the preceding claims, the compound (L) changing state when it is heated up to a temperature of between 30°C and 80°C.

4. Assembly according to any one of the preceding claims, comprising at least 0.2 cm³ of said compound, preferably between 1 and 80 cm³ of the compound.

5. Assembly according to any one of the preceding claims, the applicator comprising a non-metallic material having a density greater than or equal to 1.5 g/cm³, at least partially defining the applicator surface (9) intended to come into contact with the region to be treated.

6. Assembly according to Claim 5, the material being a stone or glass.

7. Assembly according to any one of the preceding claims, the applicator comprising a material at least partially defining an applicator surface (9) intended to come into contact with the region to be treated and having a thermal conductivity greater than or equal to 1 Wm⁻¹K⁻¹, preferably 40 Wm⁻¹K⁻¹.

8. Assembly according to any one of Claims 1 to 7, the applicator comprising a material having a specific heat capacity greater than or equal to 500 Jkg⁻¹K⁻¹, preferably 1000 Jkg⁻¹K⁻¹, more preferably 2000 Jkg⁻¹K⁻¹.

9. Assembly according to any one of the preceding claims, the applicator surface (9) being at least partially defined by a material having a thermal inertia greater than or equal to 1000 Jm⁻²K⁻¹s^{-1/2}, preferably greater than or equal to 5000 Jm⁻²K⁻¹s^{-1/2}, more preferably 10 000 Jm⁻²K⁻¹s^{-1/2}.

10. Assembly according to any one of Claims 1 to 9, the applicator having a mass greater than or equal to 15 g.

11. Assembly according to any one of Claims 1 to 10, the applicator having a gripping surface (10) at least partially defined by a material having a thermal conductivity less than or equal to 1 Wm⁻¹K⁻¹, preferably less than or equal to 0.5 Wm⁻¹K⁻¹, or even less than or equal to 0.1 Wm⁻¹K⁻¹.

12. Assembly according to any one of Claims 1 to 11, the applicator being designed to be fixed in a removable manner to the container.

13. Assembly according to any one of Claims 1 to 11, the substrate being designed to be fixed in a removable manner to the applicator.

14. Assembly according to any one of Claims 1 to 12, the applicator and the container being contained in a common packaging device.

15. Assembly according to any one of Claims 1 to 14, the applicator being at least partially magnetic.

16. Assembly according to any one of Claims 1 to 15, the applicator comprising a flexible lip intended to come into contact with the region to be treated.

17. Assembly according to Claim 12, the applicator comprising a passage enabling the composition to be dispensed when the applicator is mounted on the container.

18. Assembly according to any one of the preceding claims, the applicator comprising a removable vibrator.

19. Assembly according to any one of the preceding claims, the applicator comprising a removable electrode connected to an electrical power source.

20. Assembly according to any one of the preceding claims, the applicator having an applicator surface at least partially defined by a removable piece (95), in particular made at least partially of an absorbent material.

21. Assembly according to Claim 1, the applicator comprising, between the cavity (12) and an applicator surface (9) to be brought into contact with the region to be treated, a wall having a smaller thickness (eₘᵢₙ) less than or equal to 50 mm, in particular between 0.1 mm and 50 mm, preferably less than or equal to 1 mm, more preferably less than or equal to 0.5 mm.

22. Assembly according to any one of the preceding claims, the applicator being designed, when heated up to 50°C and applied to the skin, to retain a temperature of greater than or equal to 30°C, in particular at the applicator surface, for 1 min, preferably 15 min or 30 min.

23. Assembly according to any one of the preceding claims, the applicator not comprising compounds that react together in an exothermic reaction.

24. Assembly according to any one of the preceding claims, the applicator having no electrical power source or means for connecting to an electrical power source.

25. Assembly according to any one of the preceding claims, comprising a safety cell designed to hold the applicator when it is placed in a microwave oven.

26. Assembly according to any one of the preceding claims, the applicator comprising a thermochromic indicator.

27. Assembly according to any one of the preceding claims, the cavity being bounded by a wall of non-constant thickness.

28. Assembly according to Claim 11, the applicator surface (9) and the gripping surface (10) being defined respectively by two parts assembled one on the other.

29. Cosmetic, non-therapeutic method for treating at least one region of the human body, comprising the following steps:
a) heating up an applicator (3) of an assembly as defined in any one of the preceding claims to a temperature greater than or equal to 30°C, and then
b) loading the applicator thus heated up with a cosmetic composition (P) at a temperature closer to room temperature than that of the applicator and having a difference of at least 5°C from the latter, and
applying the composition by using the applicator, or
c) before or after step a), applying at least one cosmetic composition to the region to be treated and, after step a), bringing the applicator (3) thus heated up into contact with the region to be treated.

30. Method according to Claim 29, comprising the application of a substrate (52) carrying the composition to the region to be treated, the applicator being brought into contact with the substrate thus applied.

31. Method according to one of Claims 29 and 30, the applicator being heated up by being put into a microwave oven.

## Patentansprüche

1. Einheit, die umfasst:
- eine kosmetische oder dermatologische Zusammensetzung, die in einem Behälter oder Substrat enthalten ist,
- einen Applikator (3), der von dem Behälter oder Substrat getrennt werden kann und der eine Applikationsoberfläche (9) aufweist, die nicht drehbar ist, für die Behandlung eines Hautbereichs, umfassend mindestens ein Material, das ein solches thermisches Verhalten aufweist, dass es, wenn es auf eine Temperatur von 50 °C erhitzt wird, beim Kontakt der Applikationsoberfläche mit der Haut über einen Zeitraum von 15 s keine thermische Verletzung der Haut hervorruft, wobei die Applikationsoberfläche über einen Zeitraum von mindestens 30 s nach dieser Applikation eine Temperatur von 30 °C oder darüber hält,
wobei der Applikator mindestens einen Hohlraum (12) umfasst, der eine flüssige Verbindung (L) enthält, die auf eine Temperatur von 30 °C oder darüber und 80 °C oder darunter erhitzt werden kann, wobei der Hohlraum geschlossen ist.

2. Einheit nach Anspruch 1, wobei der Applikator (3) dafür vorgesehen ist, in einem Mikrowellenofen erhitzt zu werden.

3. Einheit nach einem der vorhergehenden Ansprüche, wobei die Verbindung (L) den Zustand ändert, wenn sie auf eine Temperatur erhitzt wird, die im Bereich von 30 °C bis 80 °C liegt.

4. Einheit nach einem der vorhergehenden Ansprüche, die mindestens 0,2 cm³ dieser Verbindung und besser 1 bis 80 cm³ der Verbindung enthält.

5. Einheit nach einem der vorhergehenden Ansprüche, wobei der Applikator ein nichtmetallisches Material mit einer Dichte umfasst, die größer als oder gleich 1,5 g/cm³ ist, das mindestens teilweise die Applikationsoberfläche (9) definiert, die dafür vorgesehen ist, mit dem zu behandelnden Bereich in Kontakt zu kommen.

6. Einheit nach Anspruch 5, wobei das Material ein Stein oder aus Glas ist.

7. Einheit nach einem der vorhergehenden Ansprüche, wobei der Applikator ein Material umfasst, das mindestens teilweise eine Applikationsoberfläche (9) definiert, die dafür vorgesehen ist, mit dem zu behandelnden Bereich in Kontakt zu kommen, und das eine Wärmeleitfähigkeit aufweist, die größer als oder gleich 1 Wm⁻¹K⁻¹, besser 40 Wm⁻¹K⁻¹, ist.

8. Einheit nach einem der Ansprüche 1 bis 7, wobei der Applikator ein Material mit einer massebezogenen spezifische Wärme größer als oder gleich 500 J kg⁻¹ K⁻¹, besser 1 000 J kg⁻¹ K⁻¹, noch besser 2000 J kg⁻¹ K⁻¹, aufweist.

9. Einheit nach einem der vorhergehenden Ansprüche, wobei die Applikationsoberfläche (9) mindestens teilweise durch ein Material definiert ist, das eine thermische Trägheit größer als oder gleich 1000 Jm⁻² K⁻¹ s^{-1/2}, besser größer als oder gleich 5000, noch besser 10 000 Jm⁻²K⁻¹s^{-1/2}, aufweist.

10. Einheit nach einem der Ansprüche 1 bis 9, wobei der Applikator eine Masse von 15 g oder darüber aufweist.

11. Einheit nach einem der Ansprüche 1 bis 10, wobei der Applikator eine Greifoberfläche (10) aufweist, die mindestens teilweise durch ein Material definiert ist, das eine Wärmeleitfähigkeit kleiner als oder gleich 1 W m⁻¹K⁻¹, besser kleiner als oder gleich 0,5 W m⁻¹K⁻¹, sogar kleiner als oder gleich 0,1 Wm⁻¹K⁻¹, aufweist.

12. Einheit nach einem der Ansprüche 1 bis 11, wobei der Applikator so angeordnet ist, dass er ablösbar auf dem Behälter befestigt ist.

13. Einheit nach einem der Ansprüche 1 bis 11, wobei das Substrat so angeordnet ist, dass es ablösbar auf dem Applikator befestigt ist.

14. Einheit nach einem der Ansprüche 1 bis 12, wobei der Applikator und der Behälter in einer gemeinsamen Vorrichtung zum Verpacken enthalten sind.

15. Einheit nach einem der Ansprüche 1 bis 14, wobei der Applikator mindestens teilweise magnetisch ist.

16. Einheit nach einem der Ansprüche 1 bis 15, wobei der Applikator eine flexible Lippe umfasst, die dafür vorgesehen ist, mit dem zu behandelnden Bereich in Kontakt zu treten.

17. Einheit nach Anspruch 12, wobei der Applikator einen Durchlass umfasst, der es ermöglicht, die Zusammensetzung zu verteilen, wenn der Applikator auf dem Behälter montiert ist.

18. Einheit nach einem der vorhergehenden Ansprüche, wobei der Applikator einen ablösbaren Vibrator umfasst.

19. Einheit nach einem der vorhergehenden Ansprüche, wobei der Applikator eine ablösbare Elektrode umfasst, die mit einer Stromquelle verbunden ist.

20. Einheit nach einem der vorhergehenden Ansprüche, wobei der Applikator eine Applikationsoberfläche aufweist, die mindestens teilweise durch ein ablösbares Teil (95), insbesondere teilweise aus einem absorbierenden Material, definiert ist.

21. Einheit nach Anspruch 1, wobei der Applikator zwischen dem Hohlraum (12) und einer Applikationsoberfläche (9), die mit dem zu behandelnden Bereich in Kontakt gebracht werden soll, eine Wand aufweist, die eine geringere Dicke (eₘᵢₙ) aufweist, die kleiner als oder gleich 50 mm ist, insbesondere im Bereich von 0,1 mm bis 50 mm liegt, besser kleiner als oder gleich 1 mm und noch besser kleiner als oder gleich 0,5 mm ist.

22. Einheit nach einem der vorhergehenden Ansprüche, wobei der Applikator so angeordnet ist, dass er, wenn er auf 50 °C erhitzt ist und auf die Haut aufgebracht wird, über 1 min, besser 15 min oder 30 min eine Temperatur von 30 °C oder darüber hält, insbesondere im Bereich der Applikationsoberfläche.

23. Einheit nach einem der vorhergehenden Ansprüche, wobei der Applikator keine Verbindungen enthält, die in einer exothermen Reaktion miteinander reagieren.

24. Einheit nach einem der vorhergehenden Ansprüche, wobei der Applikator keine Stromquelle oder keine Einrichtung zum Verbinden mit einer Stromquelle aufweist.

25. Einheit nach einem der vorhergehenden Ansprüche, die eine Sicherheitszelle umfasst, die so konstruiert ist, dass sie den Applikator aufnimmt, wenn dieser in einem Mikrowellenofen angeordnet wird.

26. Einheit nach einem der vorhergehenden Ansprüche, wobei der Applikator einen thermochromen Indikator umfasst.

27. Einheit nach einem der vorhergehenden Ansprüche, wobei der Hohlraum durch eine Wand mit einer nicht konstanten Dicke begrenzt ist.

28. Einheit nach Anspruch 11, wobei die Applikationsoberfläche (9) und die Greifoberfläche (10) durch zwei Teile definiert sind, die aufeinander zusammengebaut sind.

29. Verfahren zur nicht-therapeutischen kosmetischen Behandlung mindestens eines Bereichs des menschlichen Körpers, das die folgenden Schritte umfasst:
a) Erhitzen eines Applikators (3) einer Einheit, die wie in einem der vorhergehenden Ansprüche definiert ist, auf eine Temperatur von 30 °C oder darüber, dann
b) Beladen des so erhitzten Applikators mit einer kosmetischen Zusammensetzung (P) mit einer Temperatur, die näher bei der Umgebungstemperatur liegt als die Temperatur des Applikators und die gegenüber dieser Temperatur einen Unterschied von mindestens 5 °C aufweist, und
Auftragen der Zusammensetzung unter Verwendung des Applikators, oder
c) Auftragen vor oder nach Schritt a) mindestens einer kosmetischen Zusammensetzung auf den zu behandelnden Bereich und nach Schritt a) Inkontaktbringen des so erhitzten Applikators (3) mit dem zu behandelnden Bereich.

30. Verfahren nach Anspruch 29, das das Auftragen eines Substrats (52), das die Zusammensetzung trägt, auf den zu behandelnden Bereich, wobei der Applikator mit dem so aufgetragenen Substrat gebracht wird.

31. Verfahren nach einem der Ansprüche 29 oder 30, wobei der Applikator erhitzt wird, indem er in einen Mikrowellenofen eingebracht wird.
